# EUROPEAN PATENT SPECIFICATION

(11) **EP 3 322 772 B1**
(45) Date of publication and mention of the grant of the patent: **22.09.2021**
(21) Application number: 16738153.2
(22) Date of filing: 12.07.2016
(51) Int. Cl.: C10G 9/20, G01N 17/00, G01N 27/82, G01N 27/87, G01N 33/204

(54) **METHODS FOR TESTING NON- OR WEAKLY FERROMAGNETIC TEST OBJECTS**
VERFAHREN ZUR PRÜFUNG NICHT- ODER NUR SCHWACH FERROMAGNETISCHER PRÜFOBJEKTE
PROCÉDÉS POUR TESTER DES OBJETS TESTS NON OU FAIBLEMENT FERROMAGNÉTIQUES

(30) Priority: 13.07.2015 EP 15176382; 01.02.2016 EP 16153599
(43) Date of publication of application: 23.05.2018
(73) Proprietor: Basell Polyolefine GmbH, 50389 Wesseling (DE)
(72) Inventor: LING, Antonio, 50354 Huerth (DE)
(74) Representative: LyondellBasell
(86) International application number: PCT/EP2016/066472
(87) International publication number: WO 2017/009309

(56) References cited:
- EP-A1- 2 207 007
- WO-A1-2004/023133
- WO-A1-2009/138503
- DE-A1- 10 150 762
- GB-A- 1 562 444
- US-A- 3 761 804
- US-A- 4 733 178
- US-A- 5 006 799
- US-A1- 2010 171 493
- Kucora Istvan: "PYROLYSIS FURNACE TUBE DAMAGING AND INSPECTION", ACTA TEHNICA CORVINIENSIS Bulletin of Engineering, vol. VII, no. 3 1 October 2014 (2014-10-01), pages 19-24, XP055802257, Retrieved from the Internet: URL:http://acta.fih.upt.ro/pdf/2014-3/ACTA -2014-3-01.pdf [retrieved on 2021-05-06]

## Description

### FIELD

The present disclosure relates to methods of detecting carburization, nitriding and/or chromium depletion e.g. to assess for damage as a result of corrosion and/or embrittlement processes in generally non- or weakly ferromagnetic test objects, in particular objects made of austenitic stainless steel, such as coils using in cracking furnaces.

### BACKGROUND

To cleave hydrocarbons, a stream of hydrocarbon is passed through a cracking tube or coil and thermally cracked at high temperatures and pressures within the coils. Coils are typically made of high-alloy austenitic (e.g. Fe-Ni-Cr alloy) stainless steel pipes joined through welding (using e.g. a CrNi weld material).

Despite the use of high quality of alloy austenitic steel, the coils during use often over years undergo a slow, continuously progressive embrittlement and/or corrosion from the inside and on the outside. In regard to the latter, for example, the exterior of the coils often come into contact with heating gas (e.g. natural gas or high methane natural gas) used in the cracking furnace as well as gaseous combustion products contained in the exhaust stream (e.g. CO₂, CO, NOₓ), such gases can diffuse into the material coils due to the high surface temperatures and allowing for the formation of nitrides and carbonitrides, which cause embrittlement of the coil material and consequently, gradually reduce the lifespan of the coils. In regard to the former, for example, as a result of thermal stress on the coil material, carbon atoms can diffuse via the wetted interior surface of the coils into the coil material and thus also contribute to progressive embrittlement of coil material. The most important effects that can lead to damage of the coil materials during operation include among others carburizing, nitriding, internal oxidation, depletion of chromium, and stress cracking due to longitudinal elongation.

Under the influence of high temperatures and pressures, such corrosion or embrittlement can lead to cracks or tears in the coil walls, which when not discovery in time, can lead to coil rupture and failure of cracking furnace. In addition to the safety concerns, the spontaneous failure of coils during operation has enormous consequences on the production performance and costs, in terms of loss production time as well as costs of any damage resulting from the coil rupture besides the cost of recoiling. Furthermore, in the event that the cracking furnace is part of a larger production system this can in turn lead to costly standstill of the entire system.

Coils can be replaced at regular intervals. However due to the high costs involved in replacing a coil in a cracking furnace, it is preferred that the coil is replaced only when it is necessary, i.e. ideally when the corrosion and/or embrittlement of the coil material has reached a state that the coil is considered to have reach "end of life". For this reason, coils are inspected at regular intervals. Metallurgical and destructive testing can only be carried out on dismantled coils and thus are not appropriate for inspecting coils in situ. Non-destructive visual inspections including dimension analyses of the coil (e.g. determination of changes in dimensions or form), which being helpful in terms of detecting certain potential failure indicators, do not allow for recognition of damage due to chromium depletion, nitriding, and/or carburizing.

In the article "PYROLYSIS FURNACE TUBE DAMAGING AND INSPECTION", I. Kucora and L. Radovanovic, ACTA TEHNICA CORVINIENSIS, Bulletin of Engineering, Tome VII, Fascicule 3, pages 19-24, carburization is assessed with a magnet on a string dropped down a tube.

In EP 2207007 A1, the detection of a nitride layer is performed by measuring the force necessary to remove a magnet from the object under test.

In WO 2004/023133 A1, the degree of chromium depletion is determined from the resultant magnetic flux measured by a hall element.

### SUMMARY OF THE INVENTION

Accordingly, there is a need for a simple, practicable, non-destructive method that allows for an easy and/or confident assessment of chromium depletion, carburization and/or nitriding.

It has been observed that for nearly all austenitic stainless steels, the aforementioned corrosion/embrittlement processes are associated with the generation of ferromagnetic regions within the austenitic steel. Without wanting to be bound to a particular theory, it is believed that in those regions where depletion of chromium and/or dissolution of carbon and/or nitrogen have reached a particular threshold, the regions undergo a structure and/or phase change from a non-magnetic austenitic structure to a structure that is ferromagnetic structure so that the original non- or weakly ferromagnetic austenitic steel coil material now includes regions therein that exhibit ferromagnetism.

Surprisingly it has been found that high strength permanent magnets, such as a magnet having a magnet pull or breakaway force equal to or greater than 75 N, will "react" to slight amounts or regions of ferromagnetism in the material making up to the coil to such an extent that measurements thereof may be practicably accomplished, the results of which can be in turn correlated to the presence and/or the extent of damage resulting from corrosion and/or embrittlement processes.

For example, it is has been found that when a neodymium disk magnet having a specified magnet pull or breakaway force of 75N or higher is placed into contact with an exterior surface of a new (i.e. unused) coil, it will not adhere or will only adhere slightly to the coil such that measured force needed to detach the magnet is minimal. In contrast, when the magnet is placed into contact with an end-of-life or ruptured coil in particular on the exterior surface near the corroded area or the rupture, the measured force needed to detach the magnet from the surface of the coil is much higher. Similarly it has been observed that when such a magnet is held fixedly in close proximity with an exterior surface of a coil, the measured attractive pull of the magnetic towards the coil is non-existent or minimal when the coil is new and is much higher when the coil has reached end-of-life or has ruptured.

Accordingly the extent of magnetization and thus in turn the extent of corrosion and/or embrittlement of a coil can be advantageously detected and/or assessed through a simple measurement of the force needed to pull a strong permanent magnet off the coil surface (referred to in the following as adhesive force) or the strength of the pull of the strong permanent magnet towards the coil surface (referred to the following attractive force) using high quality force measuring devices that are commercially readily available, such as a tension force gauges. Such an assessment is advantageous in that besides providing an on-the-spot assessment of a measured coil based on a single non-destructive measurement, trend analyses of a coil over its lifetime can be easily made through regular, non-destructive measurements of the coils in situ and by applying standard conditions and magnets and locations on the coils, thus further facilitating assessment of whether a coil needs replacement. In addition such long-term data collection of coils in situ can favorably allow for better understanding of corrosion/embrittlement trends within a particular cracking furnace, e.g. which coils and at which coil position undergo more rapid corrosion and/or embrittlement. Furthermore, such measurements conducted under standardized conditions with standard magnet probes can advantageously facilitate a calibration of a particular adhesive force or attractive force value to an extent of magnetization and thus embrittlement/corrosion of a particular coil alloy- thus facilitating the recognition of the appropriate, optimal time for coil replacement in terms of the particular alloy material or particular coil.

Accordingly, one aspect of the present disclosure is the provision of a method of detecting carburization, nitriding and/or chromium depletion in a generally non- or weakly ferromagnetic coil for cracking hydrocarbons according to independent claim 1, the method comprising the steps:
a) applying a permanent magnet having a magnet pull force equal to or greater than 75N to a surface of the coil;
b) measuring the force required to detach the magnet from the coil; and
c) correlating the result to the presence and/or the extent of damage resulting from corrosion and/or embrittlement processes.

A second aspect of the present disclosure is the provision of a method of detecting carburization, nitriding and/or chromium depletion in a generally non- or weakly ferromagnetic coil for cracking hydrocarbons according to independent claim 6, the method comprising the steps:
a) positioning a permanent magnet having a magnet pull force equal to or greater than 75N at a fixed distance from a surface of the coil, wherein the surface of the magnet facing towards the surface of the coil is the working surface of the magnet and wherein the working surface of the magnet is not in direct or indirect contact with the surface of the coil;
b) measuring the force in which the magnet is being attractively pulled towards the surface of the coil; and
c) correlating the result to the presence and/or the extent of damage resulting from corrosion and/or embrittlement processes.

Methods described herein are, according to the present invention, dedicated to the measurements of furnace coil or a part of furnace coil such as cracking coil or a part of cracking coil, the coil comprising preferably austenitic stainless steel. In regard to the latter two, this holds especially true since the methods described herein can be easily performed in conduit systems in situ (e.g. cracking coil system in a furnace) where the surface being measured is a portion of the exterior surface of the cracking coil.

The drawings and detailed description are to be regarded as illustrative in nature and not restrictive.

### BRIEF DESCRIPTION OF FIGURES

The following figures illustrate exemplary favorable embodiments of the subject matter disclosed herein. The claimed subject matter may be understood by reference to the following description taken in conjunction with the accompanying figures, in which like reference numerals identify like elements, and in which:
Figure 1 shows a front view of an exemplary system, not forming part of the present invention, suitable for use in measuring adhesive force using methods in accordance with the first aspect of the present disclosure;
Figure 2a shows a front view of an exemplary system, not forming part of the present invention, suitable for use in measuring attractive force using methods in accordance with the second aspect of the present disclosure;
Figure 2b show a perspective view of the exemplary system shown in Figure 2a showing the working surface of the magnet;
Figure 3a shows a front view of a further exemplary system, not forming part of the present invention, suitable for use in measuring attractive force using methods in accordance with the second aspect of the present disclosure; and
Figure 3b shows a plane view of the further exemplary system, not forming part of the present invention, shown in Figure 3a showing the working surface of the magnet.

### DETAILED DESCRIPTION

As mentioned above, one aspect of the present invention is a method of detecting carburization, nitriding and/or chromium depletion in a generally non- or weakly ferromagnetic coil for cracking hydrocarbons according to independent claim 1.

Herein and in regard to all methods described herein, the surface of the magnet doing the work (i.e. the surface of the magnet facing towards the surface of the coil) is referred to as the working surface of the magnet. Typically the working surface is planar.

Favorably, the step of measuring the adhesive force further includes applying a gradually increasing force such that the magnet is being pulled away from the surface of the coil, the force being applied in a direction perpendicular to the working surface of the magnetic and through the center of force of the magnet and continuing application of the force until the magnet detaches from the surface of the coil.

In regard to the center of force of a magnet, for symmetrical magnets, the center of force coincides with its center of gravity. For non-symmetrical magnets, the center of force can be identified by testing procedures known in the art.

The applied force to detach the magnet may be applied in any suitable manner, e.g. manually, pneumatically, hydraulically, or electromechanically. Furthermore the force is applied onto the magnet or onto the coil, where then the coil or magnet, respectively, is immobile. In the embodiments of testing coils e.g. in a cracking furnace, generally the applied force to detach the magnet is applied to the magnet because the coils are fixed in position within the furnace.

It will be appreciated for coils which are purely non-ferromagnetic, the working surface of the magnet will not adhere to the surface of the coil. For such cases, the measure of force required to detach the magnet from the coil (adhesive force) can be defined as zero. It has been generally found that non- or weakly ferromagnetic coils, such as austenitic stainless steel coils, in their original state will typically exhibit a measured adhesive force less than one Newton relative to a magnet working surface area of 4.335 cm² (i.e. less than 2.3 mPa in terms of the measured force per area). For such objects containing e.g. regions of higher ferromagnetism within the object due to e.g. the damaging effects of carburization, nitriding and/or chromium depletion, the measured adhesive force will be higher, whereby the resulting value of the measured adhesive force will proportional to the magnitude of ferromagnetic content in the coil. The methods described herein accordingly refer to structural changes within the coils and not to modification on the surface or within surface layers of the coils.

Favorably in methods of measuring adhesive force, the working surface of the magnet is placed directly in contact with the surface of the coil. For the sake of completeness, it should be mentioned that non-magnetic spacers of a known or specified thickness may be optionally used between the working surface of the magnet and the surface of the coil to create what is termed in the literature as "gap" or "air-gap" between the two named surfaces. Here in essence a sandwich configuration is provided - coil/spacer(s)/magnet where then the working surface is in indirect contact with the coil surface via the intermediate spacer(s). Since the magnet is adhered to the test object via the intermediate non-magnetic spacer(s), the force needed to detach the magnet will be lower than the force that would be needed to detach the magnet when it is directly adhering onto the surface of the coil. Because the primary objection of the methods described herein are to detect relatively small amounts or regions of ferromagnetism in a generally non- or weakly ferromagnetic coil and thus assess for troublesome corrosion/embrittlement damage, generally it is favorable not to use non-magnetic spacers and have the working surface of the magnet in direct contact with the coil surface.

In this regard, it is to be noted that the protective layer, e.g. nickel plating or an epoxy resin coating or a combination thereof, having a thickness of 50 micron or less (typically between 12 and 40 micron) that are typically found on the outer surfaces of commercial magnets are not to be understood to be a spacer. This holds true for a number of reasons, one of which is that the magnet pull force of a magnet is given in terms of the magnet as supplied with its protective plating and/or coating. Moreover for ease in measurement and performance of the methods described herein (i.e. both the adhesive and attractive force methods), for magnets having such a protective plating and/or coating, the exterior surface of the plated and/or coated magnet on the working end of the magnet (i.e. that end faces towards such test object surface) is deemed as the working surface. In regard to the attractive force measurements described herein, this means that the distance between the deemed working surface of the magnet and the coil surface is the specified distance.

Methods described herein for measuring attractive force purposively use a free or unoccupied gap between the working surface of the magnet and the surface of the coil. Moreover, the second aspect of the present invention provides a method of detecting carburization, nitriding and/or chromium depletion in a generally non- or weakly ferromagnetic coil for cracking hydrocarbons according to independent claim 6. This method is advantageous in that simply the attractive force is measured and the application of an applied force is not needed, facilitating the use of the method in-situ and under tight spatial constraints.

Hereto for the sake of the completeness, it should be mentioned that a non-magnetic spacer of a specified thickness may be optionally used at a location between the working surface of the magnet and coil surface so long as the working surface of the magnet is still not in direct or indirect contact with the coil surface, i.e. so long as there is still free and an unoccupied gap located somewhere between the respective surfaces. For the same reasons as outlined above in regard to adhesive force measurements, typically it is favorable not to use non-magnetic spacers in attractive force measurements.

For ease in performance and to further facilitate detection of minimal levels of ferromagnetism, it has been found advantageous to position the working surface of the magnet and the surface of the coil in close proximity. Favorably, the fixed distance between of the working surface of the magnet and the surface of the coil is in the range from 0.05 mm up to and including 6 mm. More favorably, the distance is equal to or less than 4.5 mm, even more favorably equal to or less than 3 mm, and most favorably, equal to or less than 2 mm. Alternatively or in addition, the distance is more favorably equal to or greater than 0.075 mm, even more favorably equal to or greater than 0.1 mm, and most favorably, equal to or greater than 0.5 mm. In the event, due to the particular form of the coil surface and/or the working surface of the magnet, the height of the free gap between two surfaces is not generally equidistant over the area of the two surfaces, then the height of the lowest gap between the two surfaces is to be understood to correspond to the fixed distance.

Desirably attractive force methods are performed such that the attractive force is being measured in a direction perpendicular to working surface of the magnet and through the center of force of the magnet. Again as mentioned above, the center of force of a symmetrical magnet coincides with its center of gravity; otherwise the center of force can be determined by testing.

Methods of measuring attractive force can be performed statically, e.g. the attractive force is measured at a single location/position on the coil, or alternatively methods may be performed dynamically, e.g. the magnet is displaced over the surface of the coil while maintaining the fixed distance and while measuring the attractive force. The latter mode of operation is advantageous in that it allows for an easy and rapid scan over a portion of a larger coil, such as along a length of a coil in a cracking furnace. The velocity by which the magnet is moved over the surface of the coil can be in the range from 1 mm/s to 300 mm/s. Preferably the measuring velocity is from 10 mm/s to 100 mm/s and more preferably from 30 mm/s to 50 mm/s.

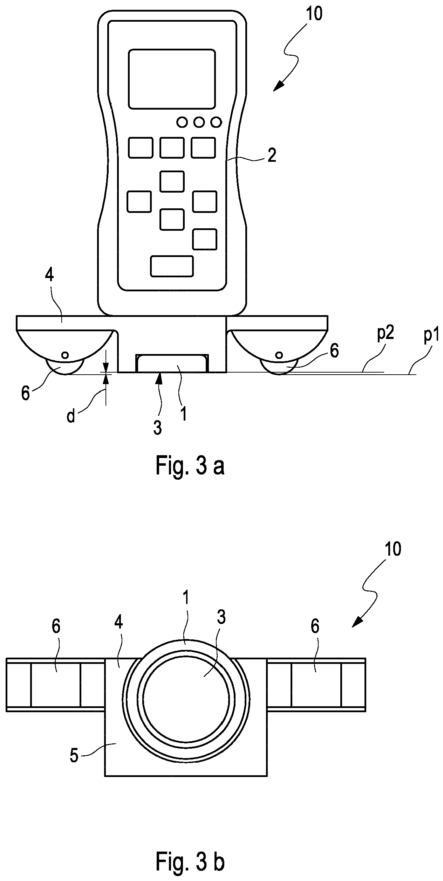

For performing the method of measuring attractive force dynamically, a system, not forming part of the present invention, is used in which the magnet is attached to a hand-held electronic force measurement device via a bracket which includes wheels or rollers, preferably two or more wheels or rollers which are arranged to lie on a straight line, and most preferably two wheels or rollers. Preferably, the most exterior surfaces of the wheels define a plane (p1) and the working surface of the magnet defines a second plane (p2) where the two planes are parallel or essentially parallel and spaced apart having a distance in the range from 0.05 mm up to and including 6 mm. More favorably, the distance is equal to or less than 4.5 mm, even more favorably equal to or less than 3 mm, and most favorably, equal to or less than 2 mm. Alternatively or in addition, the distance is more favorably equal to or greater than 0.075 mm, even more favorably equal to or greater than 0.1 mm, and most favorably, equal to or greater than 0.5 mm. An example for such a system for dynamically measuring attractive force is shown in Figure 3.

A further aspect of the present disclosure is a system for measuring magnet force, said system not forming part of the present invention, comprising a permanent magnet having a magnet pull force equal to or greater than 75N, wherein the magnet is attached to a hand-held electronic force measurement device via a bracket which includes two wheels or rollers.

Magnet pull force of permanent magnets used in the methods described herein may be determined by the standard provided the Magnet Distributors and Fabricators Association (MDFA) and published under the designation "MDFA Pull Test Standard, Standard MDFA 101 95, Test Method for Determining Breakaway Force of a Magnet"; the method being carried out at 23°C, without an optional spacer and using a mild steel test plate made of 1018 cold rolled steel (see referenced ASTM A794) and having a surface roughness of 63 ± 5 micro-inches (1.60 ± 0.13 micrometer), flatness within 0.001 inch (25.4 micrometer) over the contact surface area and a thickness such that the plate remains unsaturated by the magnetic flux of the magnet being tested (i.e. a measured flux density on the surface of the plate opposite to the surface the magnet is in contact with is less than 5 Gauss).

For ease in performance and to further facilitate detection of ferromagnetism, favorably permanent magnets used in the methods described herein have a magnet pull force equal to or greater than 90N, more favorably equal to or greater than 110N, even more favorably equal to or greater than 125N, and most favorably equal to or greater than 150N. Generally speaking, permanent magnets having a magnet pull force of 1000N or even higher may be used. However in terms of safety considerations and the dangers associated with extremely strong magnets, favorably permanent magnets in the methods described herein have a magnet pull force equal to or less than 750N, more favorably equal to or less than 600N and most favorably equal to or less than 500N.

In terms of compactness and to further facilitate e.g. working under tight spatial constraints, favorably permanent magnets used in the methods described herein are rare earth magnets, more favorably samarium or neodymium-containing magnets, and most favorably samarium cobalt alloy magnets or neodymium, iron and boron alloy magnets.

Desirably, the magnet has a size such that during measurement it does not extend beyond the outer boundaries of test object being measured. Favorably the surface area of working surface of the magnet is selected such that when the magnet is placed against the surface of the coil, the complete surface area of the working surface of the magnet can make contact to and/or faces towards the coil being measured.

For example in regard to planar test objects or test objects having a portion or portions that are substantially planar, said objects not being used in the methods of the present invention, desirably the surface area of working surface of the magnet is selected such that when the magnet is placed against the surface of the test object, the magnet can make contact over the complete surface area of its working surface to the test object being measured. In regard to the performance of attractive force measurements, this means that when the magnet positioned at the fixed distance from the test object surface, the working surface of the magnet over its complete surface area is generally (i.e. on a macroscopic level) equidistant to the surface of the test object.

In regard to non-planar test objects, e.g. test objects whose test surface has a form that is concave or convex relative to a cross-section thereof, desirably the surface area of working surface of the magnet is selected such that when the magnet is placed against the surface of the test object, the magnet can make contact over a portion of the surface area of its working surface to the surface of the test object being measured, while the remaining portion of the surface area of the magnet working remains spaced apart from the surface of the test object at a distance of no greater than 1.5 mm (more favorably no greater than 0.75 mm, most favorably no greater than 0.5 mm). For example, when a magnet having a planar working surface is placed onto the exterior surface of a tubular test object (a furnace coil such as a cracking coil) for an adhesive force measurement, typically at least a central portion of the magnet working surface will be in direct contact with the test object surface, while the remaining portions of magnet working surface will be spaced apart from the test object surface at height of 1.5 mm (more favorably 0.75 mm, most favorably 0.5 mm) or lower (i.e. down to zero) In regard to the performance of attractive force measurements, as indicated above, in the event the height of the free gap between magnet and test object surfaces is not generally equidistant, the lowest gap between the two surfaces is to be understood to correspond to the fixed distance. For example, when a magnet having a planar working surface is positioned at a fixed distance from the exterior surface of a tubular test object (a furnace coil such as a cracking coil), the height of the gap between the test object surface and the magnet working surface at the center of the magnet ("gap-height-at-center") would typically represent the lowest gap and thus the fixed distance, and favorably the height of the gap between the test object surface and the remaining portions of magnet working surface is equal to the gap-height-at-center up to a value corresponding to the gap-height-at-center plus 1.5 mm (more favorably up to the gap-height-at-center plus 0.75 mm, most favorably up to gap-height-at-center plus 0.5 mm). It will be appreciated that for both types of measurements on such non-planar objects, the working surface of the magnet is desirably applied to or positioned relative to the non-planar surface of the test object such that the normal adhesive or attractive force is being measured during the testing.

For example, taking into account typical outer diameters (70 to 300 mm) and thus typical curvature of coils in cracking furnaces, it has been found favorable to use magnets having working surfaces, where the surface area is equal to or less than 10,000 mm², more favorably equal to or less than 8,000 mm², even more favorably equal to or less than 5,000 mm² and most favorably equal to or less than 3,000 mm². Within this favored range, magnets having very small working surface areas (e.g. 3 mm² or less) may be used, but in terms of practicality and efficiency in terms of measurement and assessment generally it is favorable to use magnets having a working surface area of at least 35 mm² or greater. Moreover, it is generally favorable to select a magnet having a working surface with a high surface area (e.g. the highest possible surface area or one approaching the highest possible surface area) having regard to suitability for the particular object being tested, i.e. the form and the dimension particular test surface and/or having regard to the spatial configuration of the system or environment where the measurements are to be performed.

Accordingly, a further aspect of the present disclosure is the provision of a method of monitoring the conditions of cracking coils in a furnace, according to the dependent claim 14, comprising repeatedly detecting on the cracking coils located in situ in the furnace carburization, nitriding and/or chromium depletion by a method comprising the steps: a) applying a permanent magnet having a magnet pull force equal to or greater than 75N to a surface of the cracking coil and b) measuring the force required to detach the magnet from the cracking coil or by a method comprising the steps: a) positioning a permanent magnet having a magnet pull force equal to or greater than 75N at a fixed distance from a surface of the cracking coil, wherein the surface of the magnet facing towards the surface of the cracking coil is the working surface of the magnet and wherein said working surface of the magnet is not in direct or indirect contact with the surface of the cracking coil and b) measuring the force in which the magnet is being attractively pulled towards the surface of the cracking coil. The repeated measurements are preferably started bevor start-up of the cracking coils and are thereafter preferably carried out in time intervals of from 1 to 3 years, more preferably in time intervals of from 1 to 2 years, and especially in time intervals of about 1 year. Preferably these measurements are repeated over the whole lifetime of the cracking coils.

By repeatedly measuring the extent of magnetization and thus in turn the extent of corrosion and/or embrittlement of the cracking coils, trend analyses of a coil over its lifetime can be easily made, thus further facilitating assessment of whether a coil needs replacement. In addition such long-term data collection regarding cracking coils in situ, preferably over the whole lifetime of the cracking coils, allows for better understanding of corrosion/embrittlement trends within a particular cracking furnace, e.g. which coils and at which coil position undergo more rapid corrosion and/or embrittlement.

Methods described herein are particularly advantageously in that they allow for measurements on an exterior surface of a test object, in particular in tightly configured environments, such as on the exterior surface of a coil such as cracking coil in situ in a furnace.

Methods described herein are dedicated to the measurements of a furnace coil or a part of furnace coil such as a cracking coil or a part of cracking coil, the coil comprising an austenitic stainless steel.

### EXAMPLES

### Adhesive Force Measurement

The following method is used to measure the normal force required to detach a magnet having a specified magnet pull force and a specified work surface area from a surface of a coil.

A magnet having a specified magnet pull force and a planar working surface (i.e. that surface of the magnet that is used to perform the work in the method) of a specified surface area is used.

The magnet is selected such that it has a surface roughness (Ra) of 1.60 micrometer or less and a flatness within 25.4 micrometer over its working surface.

The area of the working surface is such that when the magnet is placed against the surface of the coil, the complete surface area of the working surface of the magnet can make contact to and/or faces towards the coil being measured. In addition, the surface area of working surface of the magnet is selected such that when the magnet is placed against the surface of the coil, the magnet can make contact over the complete surface area of its working surface to the surface of the coil being measured. Or alternatively, e.g. if the surface of the coil is non-planar (e.g. convex or concave in its cross-section), the surface area of working surface of the magnet is selected such that when the magnet is placed against the surface of the coil, the magnet can make contact over a portion of the surface area of its working surface to the coil being measured, while the remaining portion of the surface area of the magnet working will be spaced apart from the coil at a distance no greater than 1.5 mm (more favorably no greater than 0.75 mm, most favorably no greater than 0.5 mm). In the latter case, the magnet is applied to the surface of the coil in such a manner so that the plane defined by the working surface of the magnet is parallel to a plane tangent to the coil surface at a point directly opposite to the center point of the magnet working surface.

A device, not forming part of the present invention, which provides a measure of the force being exerted is used in conjunction with a method or a device to provide a gradually increasing force such that the magnet is being pulled away from the surface of the coil, whereby the force applied is perpendicular to the plane of the working surface of the magnet and through the center of force of the magnet (i.e. through the center of gravity of the magnet when magnet is symmetrical). It will be recognized that depending on the particular configuration and measurement set-up, the applied force may be a pulling force or a pushing force so long as it evokes a pulling away of the magnet from the surface of the coil.

(In alternative methods, a non-magnetic spacer of a specified thickness may be optionally used.)

The test is performed at 23°C according to the following:
1. The working surface of the magnet is placed flat against the surface of the coil. (If a non-magnetic spacer is being used, the spacer is placed between the magnet and coil.)
   In the event the magnet does not at all adhere to the coil, this is recorded and the testing is complete. Here the force value at which the magnet is detached from the surface may be denoted as "zero" Newton.
2. Attach the magnet to the measurement apparatus being used to measure adhesive force. It will be appreciated that this step may be performed prior to step 1.
3. Adjust the force-measuring device to zero after the set up is complete and prior to applying a force.
4. Apply a gradually increasing force to separate the magnet from the surface of the coil, continuing increasing the force until the magnet detaches from the coil surface.
5. Record the value at which the magnet detaches from the coil surface.
6. Repeat testing until three readings have been obtained which are within 10% of each other. Calculate the average of these results and use this value as the adhesive force.
7. Report adhesive force, magnet pull force, and surface area of magnet working surface, and, if the surface of the coil is non-planar, indication thereof and general dimensional information regarding the magnet working surface (e.g. circular and diameter thereof) and the coil surface (e.g. tubular coil and diameter thereof). (If applicable report the use of spacer and thickness thereof). To facilitate comparative studies, favorably the type and grade of magnet material as well as the presence and type of protective layer are reported.

### Attractive Force Measurement

The following method is used to measure the attractive force of a magnet towards a surface of a coil, the magnet having a specified magnet pull force and a specified work surface area and the working surface of magnet being positioned at a fixed specified distance to the surface of the coil.

A magnet having a specified magnet pull force and a planar working surface (i.e. that surface of the magnet that is used to perform the work in the method) of a specified surface area is used.

The magnet is selected such that it has a surface roughness (Ra) of 1.60 micrometer or less and a flatness within 25.4 micrometer over its working surface.

The area of the working surface is such that when the magnet is placed against the surface of the coil, the complete surface area of the working surface of the magnet can make contact to and/or faces towards the coil being measured. In addition, the surface area of working surface of the magnet is selected such that when the magnet is placed against the surface of the coil, the magnet can make contact over the complete surface area of its working surface to the surface of the coil being measured, so that during the performance of the test, the working surface over its complete surface area is generally (i.e. on a macroscopic level) equidistant to the surface of the coil. In other words, the length of the specified distance corresponds to the height of the unoccupied gap between the magnet working surface and the coil surface. Or alternatively e.g. in the case of a non-planar (e.g. convex or concave in its cross-section) coil surface, the surface area of working surface of the magnet is selected such that when the magnet is placed against the surface of the coil, the magnet can make contact over only a portion of the surface area of its working surface to the coil being measured, while the remaining portion of the surface area of the magnet working will be spaced apart from the coil at a distance, no greater than 1.5 mm (more favorably no greater than 0.75 mm, most favorably no greater than 0.5 mm) ("secondary distance") so that during the performance of the test, the free gap between the working surface and coil surface with the lowest height corresponds to the specified distance and the highest gap height is no greater than the sum of the specified distance and the secondary distance. In the latter case, the magnet is applied to the surface of the coil in such a manner so that the plane defined by the working surface of the magnet is parallel to a plane tangent to the coil surface at a point directly opposite to the center point of the magnet working surface.

A device, not forming part of the present invention, which provides a measure of the exerted (attractive) force of the magnet towards the coil surface is used in conjunction with a method or a device to position the working surface of the magnet at a specified fixed distance from coil surface and in an orientation such that the attractive force in a direction perpendicular to the surface of the coil and through the center of force of the magnet is being measured, and such that the working surface of the magnetic is not in direct or indirect contact with the surface of the coil.

(In alternative methods, a non-magnetic spacer of a specified thickness may be optionally used at a location between the working surface of the magnet and coil surface so long as the working surface of the magnet is still not in direct or indirect contact with the coil surface, i.e. so long as there is still free and an unoccupied gap located somewhere between the respective surfaces.)

The test is performed at 23°C according to the following:
1. The working surface of the magnet is positioned at fixed distance to the surface of the coil, the working surface of the magnet not be in direct or indirectly contact with the surface of the coil. (Alternatively if a non-magnetic spacer is being used, the spacer is placed between the magnet and coil, while assuring that the magnet working surface and the coil surface are not in direct or indirect contact with one another.)
2. Attach the magnet to the measurement apparatus being used to measure attractive force. It will be appreciated that this step may be performed prior to step 1.
3. Re-set the force-measuring device to zero after set up is complete.
4. Measuring the attractive (pulling) force of the magnet towards the surface of the coil and record the value.
5. Repeat steps 3 and 4 until three readings have been obtained that are within 10% of each. Calculate the average of these results and use this value as the attractive force.
6. Report attractive force, magnet pull force, surface area of magnet working surface and specified distance and, if the surface of the coil is non-planar, indication thereof and general dimensional information regarding the magnet working surface (e.g. circular and diameter thereof) and the coil surface (e.g. tubular test object and diameter thereof). (If applicable, report use of spacer and its thickness as well as unoccupied gap height and its position.) To facilitate comparative studies, favorably the type and grade of magnet material as well as the presence and type of protective layer are reported.

### Exemplary measurements of cracking coils

In the following examples, the coils measured were 100 mm in diameter and (originally) made of one of the following austenitic steel based materials:
>"GX45NiCrSiNbTi35-25" - a cast austenitic steel with 35% nickel, 25% chromium plus niobium, titanium and other components marketed by Schmidt + Clemens GmbH under the trade designation CENTRALLOY G 4852 Micro R;
>"GX45NiCrSiNbTi45-35"- an air melted nickel-base alloy consisting of a nickel-chromium-iron-silicon matrix with high chromium level and rare earth additions marketed by Schmidt + Clemens GmbH under the trade designation CENTRALLOY ET 45 Micro; and
>"GX10NiCrNb32-20" - an air melted iron-base alloy having low carbon and niobium content with an austenitic iron-chromium-nickel matrix marketed by Schmidt + Clemens GmbH under the trade designation CENTRALLOY G 4859.

A sintered NdFeB N35 pot magnet having a nickel-plated surface having a outer diameter of 32 mm, height 8 mm, and measured inner magnet-core diameter of 23.5 mm (surface area 433.5 mm² = 0.0004335 m²) as supplied by BR Technik Kontor under designation BMI 32-6 was used. The magnet pull force was measured and determined to be 25 kg (245.25 N). The sintered neodymium magnet grade N35 generally exhibits the following magnetic characteristics: remanence or residual induction (Br) 1.17 to 1.25 Tesla, coercivity (H_{cB}) ≥ 860 to 955 kA/m; intrinsic coercivity (H_{cJ}) ≥ 955 kA/m; maximum energy product (BHₘₐₓ) 263 to 302 kJ/m³.

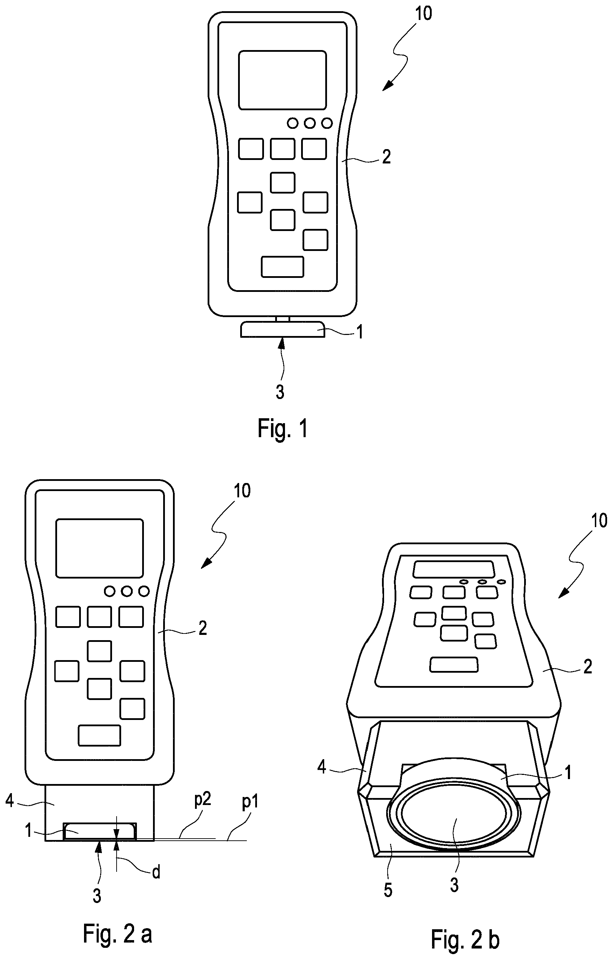

The system (10), not forming part of the present invention, used for measuring adhesive force is illustrated in Figure 1 showing a front view of the exemplary system. In particular, the pot magnet (1) described above was attached to a hand-held electronic force measurement device (2) marketed by Sauter GmbH under the designation "FL", so that during the measurements, the tension force exerted was in a direction perpendicular to the working surface (3; not visible) of the magnet and through the center of force of the magnet. In particular, for the reported coil testing, the working surface of the magnet was placed on the exterior surface of the coil being tested and after the measurement device was re-set to zero, the system was pulled manually away from cracking coil in a direction normal to the contact surface of the cracking coil, whereby the force upon detachment of the magnet from the coil was measured and recorded.

The system (10), not forming part of the present invention, used for static measurement of attractive force is illustrated in Figure 2 showing a front view (Figure 2a) and a perspective view towards the bottom (Figure 2b) of the exemplary system. In this system, the pot magnet (1) described above is attached to the hand-held electronic force measurement device (2) described above in conjunction with a bracket (4) having a mounting base (5). The magnet was attached to the measurement device such that the attractive force in a direction perpendicular to the working surface (3) of the magnet and through the center of force of the magnet was measured. As can been recognized from the Figures, the most exterior surface of the mounting base defines a plane (p1) and the working surface (3) of the magnet defines a second plane (p2), where the two planes are parallel or essentially parallel and spaced apart - the height of the space defining the specified fixed distance (d). In the particular system used, the height of the space and thus the distance was 1 mm. For ease in viewing, a greater gap height is shown in the illustration. In particular, for the reported coil testing, the bracket was oriented with its long axis along the length of the cracking coil being tested and the mounting base thereof placed on the exterior surface of the coil being tested so that 1 mm free gap was provided between the working surface of the magnet and test coil surface and after the measurement device was re-set to zero, the measurement device as allowed to measure the force in which the magnet was attractively pulled towards the surface of the test coil.

The system (10), not forming part of the present invention, used for dynamic measurement of attractive fore is illustrated in Figure 3 showing a front view (Figure 3a) and a perspective view nearly directly towards the bottom (Figure 3b) of the exemplary system. Like the exemplary system shown in Figure 2, the pot magnet (1) was attached via a bracket (4) to the hand-held electronic force measurement device (2) so that the attractive force in a direction perpendicular to the working surface (3) of the magnet and through the center of force of the magnet was measured. Here the bracket includes two wheels or rollers (6). Referring to the Figure3a, it can be seen that the most exterior surfaces of the wheels define a plane (p1) and the working surface (3) of the magnet defines a second plane (p2) where the two planes are parallel or essentially parallel and spaced apart - the height of the space defining the specified fixed distance (d). In the exemplary system used, the height of the space and thus specified distance was 1 mm. In particular, for the reported coil testing, the bracket was oriented with its long axis along the length of the cracking coil being tested and the wheels of the bracket were placed against the exterior surface of the coil so that 1 mm free gap was provided between the working surface of the magnet and test coil surface and after the measurement device was re-set to zero, the system was manually rolled along a length of the coil, while maintaining the specified fee gap and at the same length measuring the force in which the magnet was attractively pulled towards the test coil.

### Adhesive Force measurements:

The results of adhesive force measurements on cracking coils made of GX45NiCrSiNbTi35-25, which coils were located in a cracking furnace at positions at which they were heated from below, are shown in Table 1. The measurements were carried out with a magnet having a working surface of 434 mm² on groups of coils which have been used in the cracking furnace for substantially the same time. Further measurements were conducted on coils which had been replaced from such furnace. Table 2 reports similar measurements on cracking coils made of GX55CrNiSiNbTi30-30.

**Table 1. Measurement of coils made of GX45NiCrSiNbTi35-25 in a cracking furnace heated from below and such coils replaced from such cracking furnace**

| Number of Coils | Age at time of measure | Measured Force (N) | Measured Force per area (mPa) |
|---|---|---|---|
| 16 coils | 0 | <1 | <2.3 |
| 94 coils | 6 years ± 2 month | 1 - 4 | 2.3 - 9.2 |
| 96 coils* | 7 years ± 1 month | 2 - 5 plus a single 11 | 4.6 - 11.5 plus 25.3 |
| 96 coils | 10 years ± 1 month | 1 - 4 | 2.3 - 9.2 |
| | | | |
| 48 coils | deemed damaged and replaced (age at time of coil replacement: 3 years + 2 month | 17 - 31 | 39.1 - 71.3 |

| | | | |
|---|---|---|---|
| *coils in located in critical region of furnace | | | |

**Table 2. Measurement of coils made of GX55CrNiSiNbTi30-30 in a cracking furnace heated from below and such coils replaced from such cracking furnace**

| Number of Coils | Age at time of measure | Measured Force (N) | Measured Force per area (mPa) |
|---|---|---|---|
| 10 coils | 0 | <1 | <2.3 |
| 53 coils | 3 years ± 2 months | <1 - 16 | <2.3 - 36.8 |
| | | | |
| 5 coils | deemed damaged and replaced (age at time of coil replacement: 3 years + 2 month | 17 - 21 | 39.1 - 48.3 |

### Attractive Force measurements:

The results of attractive force measurements on cracking coils made of diverse austenitic steels, which coils were located in a cracking furnace at positions at which they were heated from the side and below, are shown in Table 2. The measurements were carried out with a magnet having a working surface of 434 mm² on groups of coils which have been used in the cracking furnace for substantially the same time. Further measurements were conducted on coils which had been replaced from such furnace.

**Table 3. Static attractive force measurement of 376 coils* in a cracking furnace heated from the side and a ruptured coil previously used in such cracking furnace**

| Coil ID number | Age at time of measure | Measured force (N) | Measured force per area (mPa) |
|---|---|---|---|
| No. 1 to 144 | 8.5 years | <1 | <2.3 |
| No. 145 to 197 | 2.83 years | <1 | <2.3 |
| No. 198** | 2.83 years | 19 | 43.7 |
| No. 199 | 2.83 years | < 1 | <2.3 |
| No. 200-202** | 2.83 years | 17 - 21 | 39.1 - 48.3 |
| No. 203 | 2.83 years | 11 | 25.3 |
| No. 233 to 376 | 8.75 years | <1 - 5 | <2.3 - 11.5 |
| | | | |
| Ruptured coil | age at time of rupture: 7 years + 3 month | 57 | 131.1 |

| | | | |
|---|---|---|---|
| *coils were made of either GX45NiCrSiNbTi25-25, GX55CrNiSiNbTi30-30, GX45NiCrSiNbTi45-35 or GX10NiCrNb32-20 **coils were decommissioned and replaced in light of measurement | | | |

### Comparative measurements of Adhesive versus Attractive force:

A series of measurements were performed on an approximately 325 mm long portion of two decommissioned coils, where adhesive and static attractive measurements were made at six positions along the length of the tube at 50 mm intervals starting from one end of the coil segment and dynamic attractive force measurements were made over a length of 250 mm starting from the first position and ending at the sixth position, utilizing different velocities by which the magnet was moved over the surface of the coils. The results of the measurements are provided in the following Tables 4 and 5.

**Table 4. Adhesive and attractive force measurements of a portion of a damaged and decommissioned coil originally made of GX45NiCrSiNbTi35-25 and having an age of six years at the time of replacement**

| Position | Distance from one end | Measured adhesive force (N) | Measured static attractive force (N) | Measured dynamic attractive force* (N) from 1^{st} to 6^{th} position | | |
|---|---|---|---|---|---|---|
| | | | | @ 50 mm/s | @ 100 mm/s | @ 200 mm/s |
| 1. | 50 | 16.2 | 16.1 | 18.2 | 18.1 | 17.6 |
| 2. | 100 | 15.9 | 16.1 | | | |
| 3. | 150 | 16.3 | 15.9 | | | |
| 4. | 200 | 14.6 | 14.8 | | | |
| 5. | 250 | 17.3 | 17.1 | | | |
| 6. | 300 | 18.3 | 18.1 | | | |

| | | | | | | |
|---|---|---|---|---|---|---|
| *the maximal force value observed over the measured length is reported. | | | | | | |

**Table 5. Adhesive and attractive force measurements of a portion of a damaged and decommissioned coil originally made of GX10NiCrNb32-20 and having an age of three years at the time of replacement***

| Position | Distance from one end | Measured adhesive force (N) | Measured static attractive force (N) | Measured dynamic attractive force** from 1^{st} to 6^{th} position (N) | | |
|---|---|---|---|---|---|---|
| | | | | @ 50 mm/s | @ 100 mm/s | @ 200 mm/s |
| 1. | 50 | 18,3 | 17,6 | 22.3 | 22.2 | 21.9 |
| 2. | 100 | 18,2 | 18,1 | | | |
| 3. | 150 | 22,5 | 22,1 | | | |
| 4. | 200 | 20,4 | 20,5 | | | |
| 5. | 250 | 18,3 | 18,4 | | | |
| 6. | 300 | 19,2 | 19,1 | | | |

| | | | | | | |
|---|---|---|---|---|---|---|
| *a portion of one of the measured coils from Table 3, in particular a portion of the coil No. 198. **the maximal force value observed over the measured length is reported. | | | | | | |

## Claims

1. A method of detecting carburization, nitriding and/or chromium depletion in a generally non- or weakly ferromagnetic coil for cracking hydrocarbons, the method comprising the steps: a) applying a permanent magnet having a magnet pull force equal to or greater than 75N to a surface of the coil; b) measuring the force required to detach the magnet from the coil; and
c) correlating the result to the presence and/or the extent of damage resulting from corrosion and/or embrittlement processes.

2. The method according to claim 1, wherein the coil is a conduit comprising austenitic stainless steel.

3. The method according to claim 1 or 2, wherein the coil is a furnace coil or a part of furnace coil.

4. The method according to any one of claims 1 to 3, wherein the surface of the magnet facing towards the surface of the coil is the working surface of the magnet and wherein said step of measuring further includes applying a gradually increasing force such that the magnet is being pulled away from the surface of the coil, wherein said force is being applied in a direction perpendicular to the working surface of the magnet and through the center of force of the magnet and continuing application of said force until the magnet detaches from the surface of coil.

5. The method according to any one of claims 1 to 4, wherein in said step of applying, the working surface of the magnet is placed directly in contact with the surface of the coil or the working surface is placed in indirect contact with the surface of the coil via one or more non-magnetic spacers having a known or specified thickness, said one or more spacers being located between the working surface of the magnet and the surface of the coil.

6. A method of detecting carburization, nitriding and/or chromium depletion in a generally non- or weakly ferromagnetic coil for cracking hydrocarbons, the method comprising the steps:
a) positioning a permanent magnet having a magnet pull force equal to or greater than 75N at a fixed distance from a surface of the coil, wherein the surface of the magnet facing towards the surface of the coil is the working surface of the magnet and wherein said working surface of the magnet is not in direct or indirect contact with the surface of the coil; and
b) measuring the force in which the magnet is being attractively pulled towards the surface of the coil; and
c) correlating the result to the presence and/or the extent of damage resulting from corrosion and/or embrittlement processes.

7. The method according to claim 6, wherein the distance between of the working surface of the magnet and the surface of the coil is in the range from 0.05 mm up to and including 6 mm.

8. The method according to any one of claims 6 to 7, wherein in said step of measuring, said attractive force is being measured in a direction perpendicular to working surface of the magnet and through the center of force of the magnet.

9. The method according to any one of claims to 6 to 8, wherein the method includes displacing the magnet over the surface of the coil while maintaining said fixed distance and measuring said attractive force.

10. The method according to any one of claims 6 to 9, wherein the method is carried out using a system in which the magnet is attached to a hand-held electronic force measurement device via a bracket which includes two wheels or rollers.

11. The method according to any one of the preceding claims, wherein the magnet has a magnet pull force equal to or greater than 90N, in particular equal to or greater than 110N, more particularly equal to or greater than 125N, most particularly equal to or greater than 150N;
and/or
wherein the magnet has a magnet pull force equal to or less than 750N, in particular equal to or less than 600 N, more particularly equal to or less than 500N.

12. The method according to any one of the preceding claims, wherein the magnet is a rare earth magnet, in particular a samarium or neodymium-containing magnet, more particularly a samarium cobalt alloy magnet or a neodymium, iron and boron alloy magnet.

13. The method according to any one of the preceding claims, wherein the surface area of working surface of the magnet is selected such that when the magnet is placed against the surface of the coil, the magnet can make contact over the complete surface area of its working surface to the surface of the coil being measured;
or
wherein the surface area of working surface of the magnet is selected such that when the magnet is placed against the surface of the coil, the magnet can make contact over a portion of the surface area of its working surface to the surface of the coil being measured, while the remaining portion of the surface area of the magnet working surface remains spaced apart from the surface of the coil at a distance of no greater than 1.5 mm, in particular no greater than 0.75 mm, more particularly no greater than 0.5 mm.

14. A method of monitoring the conditions of cracking coils in a furnace comprising repeatedly carrying out measurements according to a method according to any one of claims 1 to 13 on the cracking coils located in situ in the furnace.

## Patentansprüche

1. Verfahren zum Detektieren von Karbonisation, Nitrierung und/oder Chromabreicherung in einer allgemein nicht oder schwach ferromagnetischen Rohrschlange zum Cracken von Kohlenwasserstoffen, wobei das Verfahren die Schritte umfasst:
a) Applizieren eines Permanentmagneten mit einer Magnetzugkraft gleich oder größer als 75 N auf eine Oberfläche der Rohrschlange;
b) Messen der Kraft, die zum Lösen des Magneten von der Rohrschlange erforderlich ist; und
c) Korrelieren des Ergebnisses mit der Anwesenheit und/oder dem Ausmaß von Schäden, die aus Korrosions- und/oder Versprödungsprozessen resultieren.

2. Verfahren nach Anspruch 1, wobei die Rohrschlange eine Rohrleitung ist, die austenitischen rostfreien Stahl umfasst.

3. Verfahren nach Anspruch 1 oder 2, wobei die Rohrschlange eine Ofenrohrschlange oder ein Teil einer Ofenrohrschlange ist.

4. Verfahren nach einem der Ansprüche 1 bis 3, wobei die Oberfläche des Magneten, die in Richtung der Oberfläche der Rohrschlange weist, die Arbeitsoberfläche des Magneten ist, und wobei der Schritt des Messens des Weiteren das Applizieren einer allmählich ansteigenden Kraft einschließt, so dass der Magnet von der Oberfläche der Rohrschlange weg gezogen wird, wobei die Kraft in einer Richtung senkrecht zu der Arbeitsoberfläche des Magneten und durch das Kraftzentrum des Magneten hindurch appliziert wird und die Applikation der Kraft fortgesetzt wird, bis sich der Magnet von der Oberfläche der Rohrschlange löst.

5. Verfahren nach einem der Ansprüche 1 bis 4, wobei in dem Schritt des Applizierens die Arbeitsoberfläche des Magneten direkt in Kontakt mit der Oberfläche der Rohrschlange platziert wird, oder die Arbeitsoberfläche mittels einem oder mehreren nicht-magnetischen Distanzstücken mit einer bekannten oder spezifizierten Dicke in indirektem Kontakt mit der Oberfläche der Rohrschlange platziert wird, wobei das eine oder die mehreren Distanzstücke sich zwischen der Arbeitsoberfläche des Magneten und der Oberfläche der Rohrschlange befinden.

6. Verfahren zum Detektieren von Karbonisation, Nitrierung und/oder Chromabreicherung in einer allgemein nicht oder schwach ferromagnetischen Rohrschlange zum Cracken von Kohlenwasserstoffen, wobei das Verfahren die Schritte umfasst:
a) Positionieren eines Permanentmagneten mit einer Magnetzugkraft gleich oder größer als 75 N in einem festen Abstand von einer Oberfläche der Rohrschlange, wobei die Oberfläche des Magneten, die zu der Oberfläche der Rohrschlange weist, die Arbeitsoberfläche des Magneten ist, und wobei die Arbeitsoberfläche des Magneten nicht in direktem oder indirektem Kontakt mit der Oberfläche der Rohrschlange ist; und
b) Messen der Kraft, mit welcher der Magnet in anziehender Weise in Richtung der Oberfläche der Rohrschlange gezogen wird; und
c) Korrelieren des Ergebnisses mit der Anwesenheit und/oder dem Ausmaß von Schäden, die aus Korrosions- und/oder Versprödungsprozessen resultieren.

7. Verfahren nach Anspruch 6, wobei der Abstand zwischen der Arbeitsoberfläche des Magneten und der Oberfläche der Rohrschlange im Bereich von 0,05 mm bis zu und einschließlich 6 mm beträgt.

8. Verfahren nach einem der Ansprüche 6 bis 7, wobei in dem Schritt des Messens die Anziehungskraft in einer Richtung senkrecht zu der Arbeitsoberfläche des Magneten und durch das Kraftzentrum des Magneten hindurch gemessen wird.

9. Verfahren nach einem der Ansprüche 6 bis 8, wobei das Verfahren Verschieben des Magneten über die Oberfläche der Rohrschlange einschließt, während der feste Abstand aufrechterhalten wird und die Anziehungskraft gemessen wird.

10. Verfahren nach einem der Ansprüche 6 bis 9, wobei das Verfahren unter Verwendung eines Systems durchgeführt wird, bei dem der Magnet an einer handgeführten elektronischen Kraftmessvorrichtung mittels einer Halterung befestigt wird, die zwei Räder oder Walzen einschließt.

11. Verfahren nach einem der vorhergehenden Ansprüche, wobei der Magnet eine Magnetzugkraft gleich oder größer als 90 N, insbesondere gleich oder größer als 110 N, spezieller gleich oder größer als 125 N, besonders gleich oder größer als 150 N aufweist;
und/oder
wobei der Magnet eine Magnetzugkraft gleich oder kleiner als 750 N, insbesondere gleich oder kleiner als 600 N, spezieller gleich oder kleiner als 500 N aufweist.

12. Verfahren nach einem der vorhergehenden Ansprüche, wobei der Magnet ein Seltenerdmagnet ist, insbesondere ein Samarium oder Neodym enthaltender Magnet, insbesondere ein Magnet aus Samarium-Kobalt-Legierung oder ein Magnet aus Neodym-, Eisen- und Borlegierung.

13. Verfahren nach einem der vorhergehenden Ansprüche, wobei der Flächeninhalt der Arbeitsoberfläche des Magneten so gewählt wird, dass der Magnet, wenn der Magnet an der Oberfläche der Rohrschlange platziert wird, Kontakt über den gesamten Flächeninhalt seiner Arbeitsoberfläche mit der Oberfläche der zu messenden Rohrschlange aufnehmen kann;
oder
wobei der Flächeninhalt der Arbeitsoberfläche des Magneten so gewählt wird, dass der Magnet, wenn der Magnet an der Oberfläche der Rohrschlange platziert wird, über einen Anteil des Flächeninhalts seiner Arbeitsoberfläche Kontakt mit der Oberfläche der zu messenden Rohrschlange aufnehmen kann, während der verbleibende Anteil des Flächeninhalts der Magnetarbeitsoberfläche von der Oberfläche der Rohrschlange in einem Abstand von nicht mehr als 1,5 mm, insbesondere nicht mehr als 0,75 mm, spezieller nicht mehr als 0,5 mm beabstandet bleibt.

14. Verfahren zum Überwachen der Zustände von Crackrohrschlangen in einem Ofen, umfassend wiederholtes Durchführen von Messungen gemäß einem Verfahren nach einem der Ansprüche 1 bis 13 an den Crackrohrschlangen, die sich in situ in dem Ofen befinden.

## Revendications

1. Procédé de détection de la carburation, de la nitruration et/ou de l'appauvrissement en chrome dans une bobine généralement non ferromagnétique ou faiblement ferromagnétique pour le craquage d'hydrocarbures, le procédé comprenant les étapes de :
a) application d'un aimant permanent présentant une force de traction magnétique égale ou supérieure à 75 N à une surface de la bobine ;
b) mesure de la force requise pour détacher l'aimant de la bobine ; et
c) établissement d'une corrélation entre le résultat et la présence et/ou l'étendue des dommages résultant de processus de corrosion et/ou de fragilisation.

2. Procédé selon la revendication 1, la bobine étant un conduit comprenant de l'acier inoxydable austénitique.

3. Procédé selon la revendication 1 ou 2, la bobine étant une bobine de four ou une partie de bobine de four.

4. Procédé selon l'une quelconque des revendications 1 à 3, la surface de l'aimant orientée vers la surface de la bobine étant la surface de travail de l'aimant et ladite étape de mesure comprenant en outre l'application d'une force augmentant progressivement de telle sorte que l'aimant est éloigné de la surface de la bobine, ladite force étant appliquée dans une direction perpendiculaire à la surface de travail de l'aimant et à travers le centre de force de l'aimant, et la poursuite de l'application de ladite force jusqu'à ce que l'aimant se détache de la surface de la bobine.

5. Procédé selon l'une quelconque des revendications 1 à 4, où, dans ladite étape d'application, la surface de travail de l'aimant est placée directement en contact avec la surface de la bobine ou la surface de travail est placée en contact indirect avec la surface de la bobine par l'intermédiaire d'un ou plusieurs espaceurs non magnétiques présentant une épaisseur connue ou spécifiée, ledit un ou lesdits plusieurs espaceurs étant situés entre la surface de travail de l'aimant et la surface de la bobine.

6. Procédé de détection de la carburation, de la nitruration et/ou de l'appauvrissement en chrome dans une bobine généralement non ferromagnétique ou faiblement ferromagnétique pour le craquage d'hydrocarbures, le procédé comprenant les étapes de :
a) positionnement d'un aimant permanent présentant une force de traction magnétique égale ou supérieure à 75 N à une distance fixe d'une surface de la bobine, la surface de l'aimant orientée vers la surface de la bobine étant la surface de travail de l'aimant et ladite surface de travail de l'aimant n'étant pas en contact direct ou indirect avec la surface de la bobine ; et
b) mesure de la force avec laquelle l'aimant est tiré par attraction vers la surface de la bobine ; et
c) établissement d'une corrélation entre le résultat et la présence et/ou l'étendue des dommages résultant de processus de corrosion et/ou de fragilisation.

7. Procédé selon la revendication 6, la distance entre la surface de travail de l'aimant et la surface de la bobine étant dans la plage de 0,05 mm jusqu'à et y compris 6 mm.

8. Procédé selon l'une quelconque des revendications 6 à 7, où, dans ladite étape de mesure, ladite force d'attraction est mesurée dans une direction perpendiculaire à la surface de travail de l'aimant et à travers le centre de force de l'aimant.

9. Procédé selon l'une quelconque des revendications 6 à 8, le procédé comprenant le déplacement de l'aimant sur la surface de la bobine tout en maintenant ladite distance fixe et la mesure de ladite force d'attraction.

10. Procédé selon l'une quelconque des revendications 6 à 9, le procédé étant mis en œuvre à l'aide d'un système dans lequel l'aimant est fixé à un dispositif électronique portatif de mesure de force par l'intermédiaire d'un support qui comprend deux roues ou rouleaux.

11. Procédé selon l'une quelconque des revendications précédentes, l'aimant présentant une force de traction magnétique égale ou supérieure à 90 N, en particulier égale ou supérieure à 110N, plus particulièrement égale ou supérieure à 125 N, le plus particulièrement égale ou supérieure à 150 N ;
et/ou
l'aimant présentant une force de traction magnétique égale ou inférieure à 750 N, en particulier égale ou inférieure à 600 N, plus particulièrement égale ou inférieure à 500 N.

12. Procédé selon l'une quelconque des revendications précédentes, l'aimant étant un aimant aux terres rares, en particulier un aimant contenant du samarium ou du néodyme, plus particulièrement un aimant en alliage de samarium-cobalt ou un aimant en alliage de néodyme, de fer et de bore.

13. Procédé selon l'une quelconque des revendications précédentes, l'aire surfacique de la surface de travail de l'aimant étant sélectionnée de telle sorte que, lorsque l'aimant est placé contre la surface de la bobine, l'aimant peut entrer en contact sur l'aire surfacique totale de sa surface de travail avec la surface de la bobine qui est mesurée ;
ou
l'aire surfacique de la surface de travail de l'aimant étant sélectionnée de telle sorte que, lorsque l'aimant est placé contre la surface de la bobine, l'aimant peut entrer en contact sur une partie de l'aire surfacique de sa surface de travail avec la surface de la bobine qui est mesurée, tandis que la partie restante de l'aire surfacique de la surface de travail magnétique reste écartée de la surface de la bobine à une distance qui n'est pas supérieure à 1,5 mm, en particulier pas supérieure à 0,75 mm, plus particulièrement pas supérieure à 0,5 mm.

14. Procédé de surveillance des conditions de bobines de craquage dans un four, comprenant la réalisation répétée de mesures selon un procédé selon l'une quelconque des revendications 1 à 13 sur les bobines de craquage situées in situ dans le four.
